# FASCICULE DE BREVET EUROPEEN

(11) **EP 3 302 687 B1**
(45) Date de publication et mention de la délivrance du brevet: **28.04.2021**
(21) Numéro de dépôt: 16729020.4
(22) Date de dépôt: 12.05.2016
(51) Int. Cl.: A61N 1/05, A61N 1/36, A61N 5/06

(54) **DISPOSITIF POUR STIMULATION ELECTRIQUE ET OPTIQUE PROFONDE DU CERVEAU**
VORRICHTUNG FÜR TIEFE ELEKTRISCHE UND OPTISCHE HIRNSTIMULATION
DEVICE FOR DEEP ELECTRICAL AND OPTICAL BRAIN STIMULATION

(30) Priorité: 28.05.2015 FR 1554789
(43) Date de publication de la demande: 11.04.2018
(73) Titulaire: Commissariat à l'Énergie Atomique et aux Énergies Alternatives, 75015 Paris (FR)
(72) Inventeur: CHABROL, Claude, 38320 Poisat (FR); BENABID, Alim-Louis, 38240 Meylan (FR)
(74) Mandataire: Cabinet Laurent & Charras
(86) Numéro de dépôt international: PCT/FR2016/051124
(87) Numéro de publication internationale: WO 2016/189216

(56) Documents cités:
- WO-A1-2009/026382
- US-A1- 2005 070 987
- US-A1- 2010 241 100
- US-A1- 2014 074 182
- RUBEHN B ET AL: "Polymer-based shaft microelectrodes with optical and fluidic capabilities as a tool for optogenetics", ENGINEERING IN MEDICINE AND BIOLOGY SOCIETY,EMBC, 2011 ANNUAL INTERNATIONAL CONFERENCE OF THE IEEE, IEEE, 30 août 2011 (2011-08-30), pages 2969-2972, XP032319385, DOI: 10.1109/IEMBS.2011.6090815 ISBN: 978-1-4244-4121-1

## Description

### DOMAINE DE L'INVENTION

L'invention a trait au domaine des dispositifs implantables dans un cerveau humain ou animal, et plus particulièrement au domaine des dispositifs réalisant la stimulation cérébrale profonde du cerveau, notamment pour le traitement des maladies neurodégénératives telles que la maladie de Parkinson.

### ETAT DE LA TECHNIQUE

La stimulation cérébrale profonde d'un cerveau, plus communément appelée « *deep brain stimulation* » (DBS), consiste à stimuler électriquement par des impulsions de faible courant des parties du cerveau humain contrôlant les mouvements, et plus particulièrement le thalamus, le noyau sous-thalamique et/ou le *globus pallidus,* ce qui permet au patient une meilleure maîtrise de ses mouvements. Cette stimulation est utilisée avec succès dans le traitement de la maladie de Parkinson, les tremblements, des TOC ou les dystonies.

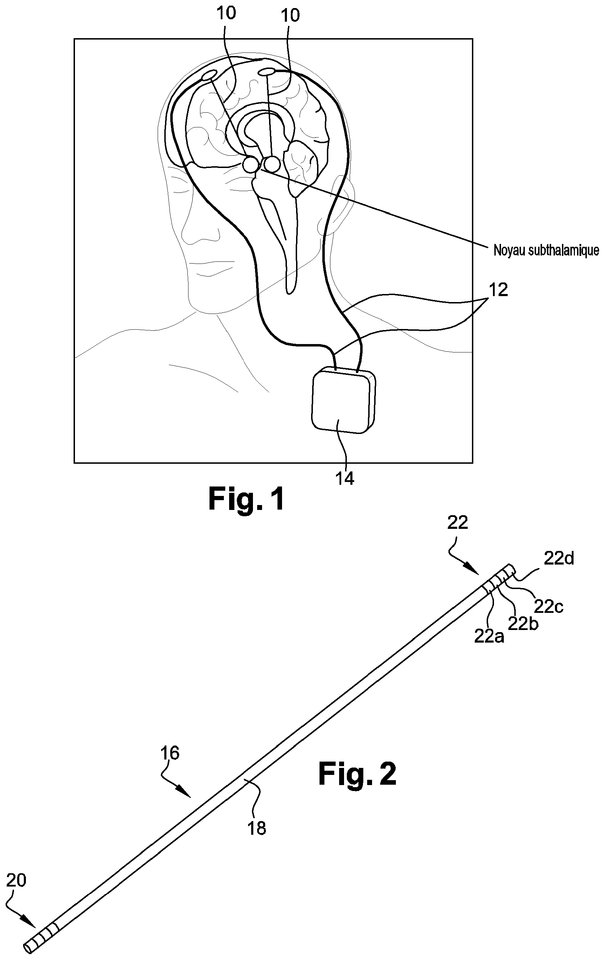

Dans la pratique, et en se référant à la figure 1, deux jeux d'électrodes **10** sont implantées dans le cerveau pour la stimulation électrique de ce dernier, chacun de ces jeux étant disposés en bout d'un corps souple longiligne en matériau biocompatible, également appelés sondes « DBS ». L'implantation d'une sonde consiste à pratiquer une ouverture dans la boite crânienne du patient, à faire pénétrer la sonde par la trépanation pratiquée jusqu'à placer son extrémité distale à l'emplacement souhaité, en s'aidant pour cela de l'imagerie cérébrale en temps réel (scanner, IRM,...) et de la reconstruction tridimensionnelle du cerveau. En raison du matériau qui la constitue et/ou de son diamètre, la sonde est usuellement très flexible et difficile à introduire dans le cerveau par une simple poussée. C'est pourquoi, la sonde est pourvue d'un canal central dans lequel le praticien introduit une tige peu flexible, appelée « raidisseur », qui aide, voire tout simplement rend possible, la progression de la sonde dans le cerveau par poussée.

Lors de cette opération, le patient est sous anesthésie locale afin de pratiquer des tests sur l'efficacité de la stimulation. Une fois les sondes en place, le patient est alors placé sous anesthésie générale, et une extension **12** de chaque sonde est logée sous le cuir chevelu et la peau du cou pour la connexion des sondes à un stimulateur électrique à pile **14** logé sous la peau du patient en position sous claviculaire.

Dans ce qui suit, le terme « distal » se rapporte à une portion ou une extrémité d'un élément, destinée à être en contact avec la zone à traiter. Le terme « proximal » se rapporte à une portion ou une extrémité de l'élément, opposée à la portion ou extrémité distale et usuellement destinée à la connexion de l'élément avec un stimulateur.

De nombreuses sondes DBS ont été ainsi conçues, par exemple par les sociétés Medscape©, Medtronics©, BostonScientic©, etc...

En se référant aux figures 2 à 4, une sonde DBS **16** comporte usuellement :
- un corps de sonde longiligne cylindrique électriquement isolant et souple **18,** d'un diamètre faible, typiquement de 1,3 mm, ce corps de sonde peut être un simple tube (MEDTRONIC) ou un tube multi lumen (Boston Scientific) ;
- un ensemble **20** d'électrodes de stimulation électrique **20a, 20b, 20c, 20d** disposées dans une portion distale du corps **18,** et
- un ensemble **22** de contacts d'alimentation **22a, 22b, 22c, 22d** disposés dans une portion proximale du corps **18.**

Les électrodes **20a, 20b, 20c, 20d,** souvent au nombre de quatre, prennent la forme de cylindres électriquement conducteurs enveloppant une surface externe du corps de sonde **18.** Ce dernier comprend par ailleurs en son centre un tube creux **26,** et vide de matière, qui s'étend sur toute sa longueur du corps **18,** et dont le diamètre est usuellement de 0,45 à 0,55mm de diamètre pour permettre l'introduction pendant la chirurgie d'un raidisseur métallique de 0,3 à 0,35mm de diamètre.

Un ensemble **28** de fils conducteurs électriquement isolés **28a, 28b, 28c, 28d** est enroulé autour du tube **26,** respectivement soudés aux électrodes **20a, 20b, 20c, 20d** et aux contacts **22a, 22b, 22c, 22d,** et noyés dans le matériau du corps de sonde **18.** Les fils **28a, 28b, 28c, 28d** ont typiquement un diamètre compris entre 100µm à 150µm

L'extrémité distale du corps de sonde **18** est en outre fermée par un bouchon hémisphérique **30** inséré dans le tube creux **26.** Ainsi, le canal central est hermétiquement fermé (hormis à l'extrémité proximale de la sonde), de sorte à éviter toute introduction de fluide dans celui-ci, mais également de sorte à éviter une contamination du cerveau via le canal central lors de l'implantation de la sonde. Les contacts **22a, 22b, 22c, 22d** sont généralement également des cylindres enveloppant une surface externe proximale du corps de sonde **18,** et respectivement connectés aux fils conducteurs **28a, 28b, 28c, 28d,** leur dimension et espacement pouvant toutefois être différents de ceux des électrodes **20a, 20b, 20c, 20d.**

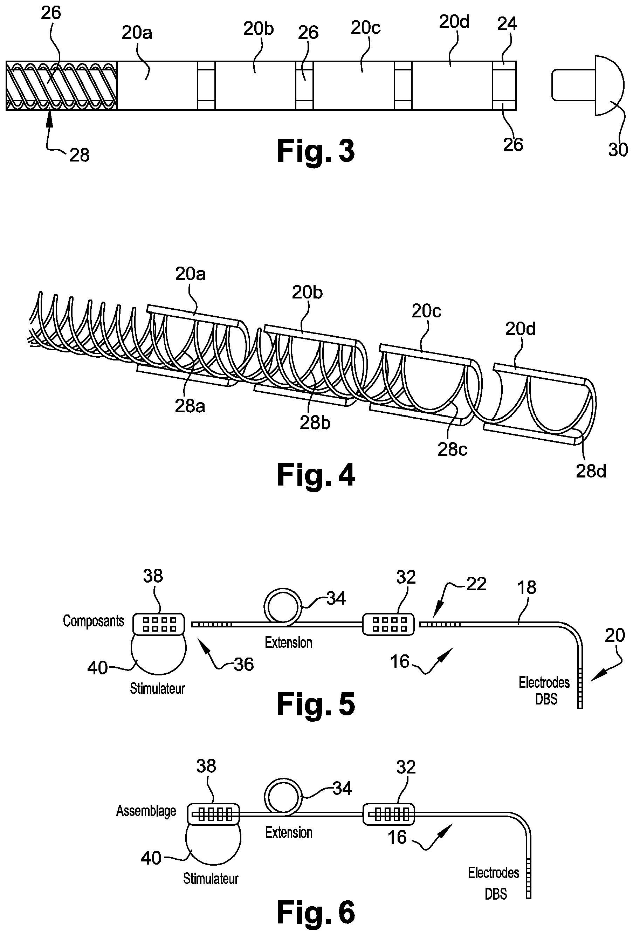

En se référant aux figures 5 et 6, la connexion électrique d'une sonde DBS **16** consiste à introduire la portion proximale de celle-ci, pourvue des contacts **22a, 22b, 22c, 22d,** dans le canal d'un boitier électrique **32,** comprenant des contacts/bornes internes qui viennent respectivement au contact des contacts **22a, 22b, 22c, 22d** de la sonde. Le boitier **32,** qui constitue une simple reprise de contacts électriques, et ne comporte donc aucun élément de commande électrique, se prolonge par rallonge filaire **34,** pourvue de contacts **36** à une portion proximale pour une connexion à une connectique **38** d'un stimulateur électrique **40.**

Parallèlement à la stimulation électrique profonde du cerveau, un intérêt croissant est porté sur la stimulation optique profonde du cerveau, notamment à l'aide d'un rayonnement dans le rouge visible ou le proche infrarouge, pour le traitement des mêmes maladies que celles traitées par la stimulation électrique, comme le décrit par exemple l'article « Red and NIR light dosimetry in the human deep brain » de A Pitzschke et al., Phys. Med. Biol. 60 2921, 2015. Cette nouvelle approche induit notamment le développement de nouvelles sondes pourvues, en position distale, de sorties optiques, généralement à base de matériaux polymères spécifiques qui doivent présenter à la fois les propriétés d'émission optique recherchées et de biocompatibilité.

La stimulation électrique ne fait que compenser le manque de dopamine en excitant la STN, mais n'a aucun effet thérapeutique sur la maladie de Parkinson qui continue à évoluer. Les résultats constatés sur les tests précliniques montrent qu'une stimulation optique apporte une protection cellulaire des cellules de la SNc dont la dégénérescence cause la maladie de Parkinson. La combinaison des deux modes de stimulations permettrait d'avoir une protection cellulaire et ainsi stopper la maladie, tout en réduisant les symptômes (akinésie, rigidité, tremblement...) en stimulant la STN chez les patients déjà dans une phase avancée de la maladie. Il n'est donc pas exclu de combiner les deux approches pour stimuler une même zone du cerveau en vue de traiter une maladie particulière, par exemple la maladie de Parkinson. Toutefois, cela suppose de multiplier le nombre de sondes implantées dans le cerveau, ce qui est difficilement envisageable. En variante, il est possible de concevoir des sondes qui comprennent chacune deux jeux d'électrodes en position distale, un jeu d'électrodes pour la stimulation électrique et un jeu d'électrodes pour la stimulation optique. Cependant, ceci suppose une zone d'émission opto-électrique importante de la sonde, et donc possiblement un traitement trop peu sélectif spatialement, outre la multiplication des fils conducteurs à prévoir dans la sonde. Il est également possible de chercher à concevoir des électrodes qui conviennent pour les deux types de stimulation, électrique et optique, et qui soient en outre biocompatibles. On conçoit cependant aisément la difficulté qu'implique le développement de telles électrodes.

Le document US2010/241100 décrit une sonde de stimulation électrique et optique des zones profondes du cerveau.

### EXPOSE DE L'INVENTION

Le but de l'invention est de proposer un dispositif de stimulation opto-électrique profonde d'un cerveau qui soit de conception simple.

A cet effet, l'invention a pour objet un dispositif selon la revendication 1 pour la stimulation profonde d'un cerveau, comprenant entre autre :
- une sonde de stimulation comprenant :
   ∘ un corps longiligne parcouru par un canal central vide de matière s'étendant entre une portion distale et une extrémité proximale de la sonde, le canal central étant ouvert à ladite extrémité proximale et la portion distale du corps étant apte à transmettre une longueur d'onde prédéterminée ;
   ∘ des électrodes fixées sur une surface externe du corps dans la portion distale de la sonde pour la stimulation électrique du cerveau ;
   ∘ des bornes électriques comprenant chacune une portion fixée sur une surface externe du corps dans la portion proximale de la sonde et connectées aux électrodes pour leur alimentation électrique;
- un boitier électrique comprenant un canal dans lequel sont logées des bornes électriques, ledit canal étant ouvert à l'une de ses extrémité pour recevoir la portion proximale de la sonde de manière à mettre en contact les bornes électriques de la sonde et les bornes électriques du boitier ; et
- une rallonge électrique fixée au boitier électrique et connectée aux bornes électriques du boitier.
- un guide d'onde apte à guider la longueur d'onde prédéterminée, logé au moins en partie dans le canal central du corps de la sonde, et s'étendant jusqu'à la portion distale de la sonde pour l'illumination du cerveau au travers de celle-ci; et
- un boitier émetteur apte à être raccordé à une extrémité proximale du guide d'onde pour l'injection dans celui-ci de la longueur d'onde prédéterminée.

En d'autres termes, l'invention se fonde sur la constatation des inventeurs que des sondes DBS de l'état de la technique peuvent être modifiées simplement pour mettre également en œuvre une stimulation optique. En effet, une sonde DBS dispose d'un canal central qui n'est pas employé pour la stimulation électrique, le matériau du corps de la sonde (e.g., du silicone, ou du polyuréthane) est usuellement transparent aux longueurs d'onde d'intérêt pour la stimulation optique, et les électrodes de stimulation électrique ne recouvrent pas la totalité de la portion distale de la sonde, notamment son extrémité, ce qui laisse donc une surface pour l'émission optique. En outre, le diamètre du canal des sondes de l'état de la technique est compatible avec le diamètre de certaines fibres optiques convenant au transport de la longueur d'onde pour la stimulation optique.

Ainsi, en introduisant une fibre optique dans le canal central d'une sonde DBS jusqu'à l'extrémité distale de celle-ci, on obtient une sonde modifiée qui, sur sa portion distale, est capable d'émettre des stimulations électriques au moyen des électrodes et une émission optique au moins par l'extrémité distale de la sonde. En outre, en cas de besoin, la fibre, qui est logée de manière amovible dans le canal central, peut être retirée ou repositionnée. De manière avantageuse, le guide d'onde est introduit une fois la sonde implantée, signifiant que le canal central conserve sa fonction première, à savoir la réception d'un raidisseur aidant à la progression de la sonde dans le cerveau.

Toutefois, la connexion électrique d'une sonde DBS de l'état de la technique se fait invariablement au moyen d'une rallonge qui obstrue le canal central, de sorte qu'en l'état, il n'est pas possible d'introduire un guide d'onde (e.g. une fibre optique) dans le canal de la sonde et de connecter ce guide d'onde.

Selon l'invention, la connectique de la sonde DBS est donc modifiée pour libérer le canal du boitier électrique en déportant la rallonge électrique fixée au boitier. Le canal du boitier étant libéré, on peut donc y introduire le guide d'onde ainsi que permettre le raccordement du guide d'onde avec une source d'émission lumineuse.

Selon des modes de réalisation, le dispositif comprend une ou plusieurs des caractéristiques suivantes :
- la portion distale du guide d'onde et la portion distale de la sonde ont une longueur comprise entre 2 millimètres et 10 millimètres ;
- la longueur d'onde est comprise entre 670 nanomètres et 1070 nanomètres, et de préférence entre 670 et 810 nanomètres, et la puissance optique émise par la portion distale du guide d'onde est inférieure ou égale à 10 milliwatts, notamment pour le traitement de la maladie de Parkinson;
- le guide d'onde est une fibre optique de type HCS (pour l'expression anglo-saxonne *« hard-clad silica* ») ayant un cœur silice de diamètre compris entre 125 micromètres et 300 micromètres, un coating (ou « clad ») de diamètre inférieure ou égale à 15 micromètres et une gaine (ou « buffer ») de diamètre inférieure ou égal à 500 micromètres, ou tout autre guide d'onde (silicone, PMMA, Silice-silice) de diamètre inférieur ou égal à 500 micromètres.

L'invention a également pour objet une extension électrique selon la revendication 5, pour une connexion avec une sonde de stimulation profonde d'un cerveau, ladite sonde comprenant entre autre un corps longiligne et des bornes électriques fixées sur une surface externe d'une portion proximale du corps de la sonde, ladite extension électrique comprenant :
- un boitier électrique comprenant :
   ∘ un canal ouvert à une première et une seconde extrémités, le canal étant apte à recevoir la portion proximale du corps de la sonde lorsque celle-ci est introduite par la première extrémité du canal ;
   ∘ des contacts électriques logés dans le canal du boitier électrique et aptes à venir au contact des bornes de la sonde lorsque la portion proximale de celle-ci est introduite dans le canal du boitier par la première extrémité du canal;
- et une rallonge électrique comprenant des conducteurs électriques raccordés aux contacts électriques du boitier, ladite rallonge électrique étant fixée au boitier sans obstruer la deuxième extrémité du canal du boitier.

Une telle extension permet de raccorder une sonde DBS en laissant libre son canal central. De manière avantageuse, cette extension est universelle puisqu'elle peut alimenter une sonde DBS, qu'elle soit associée ou non à un guide d'onde.

De manière avantageuse, un bouchon, e.g. en silicone, est employée pour obturer le canal central si ce dernier n'est pas utilisé pour loger un guide d'onde. Le bouchon peut être un septum moulé en même temps que le corps du connecteur ou une pièce qui se clips dans celui-ci.

L'invention a également pour objet un procédé de test selon la revendication 6, du fonctionnement optique d'un premier et un deuxième dispositifs de stimulation selon l'une des revendications 1 à 4, les sondes des deux dispositifs étant pré-implantées dans le cerveau avec leurs portions distales logées dans une zone du cerveau à traiter, le procédé consistant :
- à injecter un rayonnement de longueur d'onde prédéterminée dans le guide d'onde du premier dispositif par l'extrémité proximale du guide d'onde et à mesurer ledit rayonnement à l'extrémité proximale du guide d'onde du deuxième dispositif ;
- à injecter le même rayonnement dans le guide d'onde du deuxième dispositif par l'extrémité proximale du guide d'onde et à mesurer ledit rayonnement à l'extrémité proximale du guide d'onde du premier dispositif ; et
- à diagnostiquer le fonctionnement optique de l'ensemble formé des sondes et des guides d'ondes logés dans celle-ci en fonction des mesures.

Comme décrit précédemment, les sondes DBS de l'état de la technique peuvent être simplement modifiées pour mettre en œuvre également une stimulation optique, y compris les sondes DBS qui sont déjà implantées dans un patient. Il est ainsi possible de mettre à jour des sondes déjà implantées, sans avoir à les retirer, en introduisant dans leur canal central respectif des guides d'onde (e.g. des fibres optiques), en prévoyant la nouvelle connectique selon l'invention. Il convient toutefois de s'assurer que les fibres optiques ont bien été introduites et que le fonctionnement optique des sondes modifiées est conforme aux attentes. Or, par nature, un guide d'onde est également capable de capter un rayonnement par ses extrémités. Ainsi en activant l'émission optique de la première sonde (respectivement de la seconde sonde) et en désactivant l'émission de la deuxième sonde (respectivement de la première sonde), cette dernière capte donc par son extrémité distale le rayonnement de la première sonde (respectivement de la seconde sonde), le rayonnement optique ainsi collecté se propageant vers l'extrémité proximale de la sonde où il est mesuré pour être analysé. Le fonctionnement optique des sondes peut donc être diagnostiqué avec les sondes DBS toujours implantées.

### BREVE DESCRIPTION DES FIGURES

L'invention sera mieux comprise à la lecture de la description qui va suivre, donnée uniquement à titre d'exemple, et réalisée en relation avec les dessins annexés, dans lesquels :
- la figure 1 est une vue schématique de sonde, connectiques et de stimulateurs pour la stimulation électrique profonde d'un cerveau humain ;
- la figure 2 est une vue schématique en perspective d'une sonde DBS de l'état de la technique ;
- la figure 3 est une vue schématique d'une portion distale de la sonde DBS de la figure 1 ;
- la figure 4 est une vue schématique des électrodes de stimulation électrique de la sonde DBS et des fils conducteurs alimentant les électrodes ;
- les figures 5 et 6 sont des vues schématiques des différents composants utilisés pour la stimulation électrique profonde, respectivement avant leur connexion et après leur connexion ;
- la figure 7A est une vue schématique en perspective d'une sonde DBS dans laquelle est insérée une fibre optique conformément à l'invention et la figure 7B est une vue agrandie de la portion distale de la sonde DBS de la figure 7A;
- la figure 8 est une vue schématique d'un boitier de connexion électrique selon l'invention pour le raccordement à la partie proximale d'une sonde DBS pourvue de contacts électriques ;
- la figure 9 est une vue schématique illustrant un système de stimulation opto-électrique profonde d'un cerveau conforme à l'invention ;
- les figures 10A et 10B sont des vues schématiques illustrant une première et une seconde variantes intégrant la connexion d'une rallonge optique à un boitier émetteur entrant dans la constitution du système de la figure 9 ;
- les figures 11 à 15 illustrent un procédé de mise à niveau d'une paire de sondes DBS implantées chez un patient pour leur conférer la fonction de stimulation optique, la figure 14 illustrant plus particulièrement un procédé de test du bon fonctionnement optique des sondes modifiées :
- la figure 16 est une vue schématique illustrant une variante privilégiée du boitier émetteur pourvu d'une fonction de détection de rayonnement ; et
- les figures 17A et 17B illustre l'alternance d'activation et de désactivation de la fonction d'éclairement des sondes opto-électriques selon l'invention permettant de tester automatiquement le fonctionnement de celles-ci une fois le patient équipé du système selon l'invention.

### DESCRIPTION DETAILLEE DE L'INVENTION

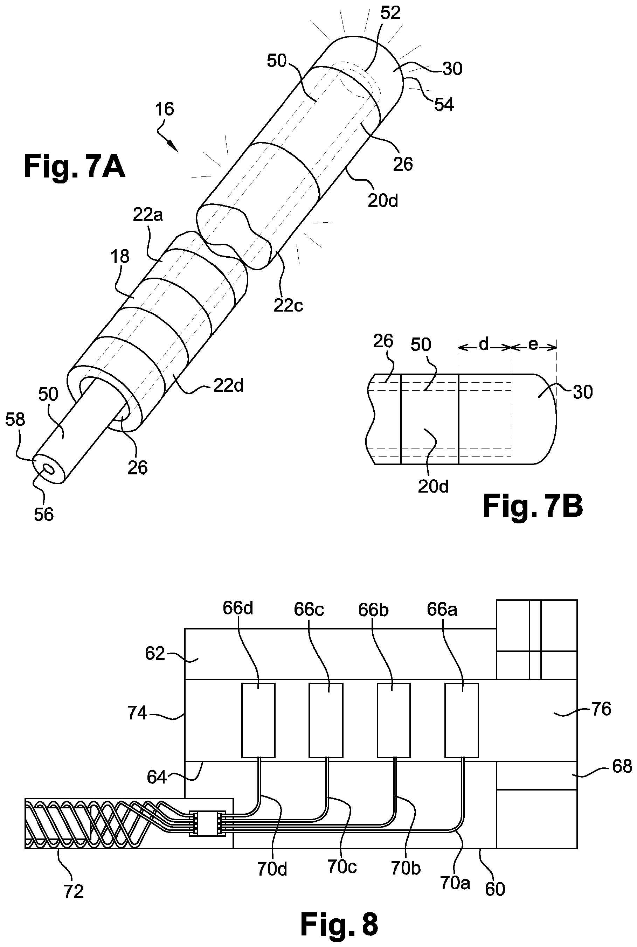

En se référant aux figures 7A et 7B, une sonde DBS **16,** par exemple celle décrite en relation avec les figures 2 à 4, comprend un corps **18** et un capot hémisphérique **30** réalisés en un matériau transparent à un rayonnement d'intérêt, c'est-à-dire laissant passer au moins 95% du rayonnement, par exemple transparent à une longueur d'onde rouge ou du proche infrarouge. Par exemple, le corps **18** est constitué de polyuréthane, de silicone, ou d'époxy qui présente un taux de transmission optique élevé du rouge et du proche infrarouge tout en présentant un indice de réfraction (de 1,43 pour le silicone par exemple) proche de celui du liquide cérébro-spinal (égal à 1,38).

La sonde **16** est en outre associée à une fibre optique **50** introduite de manière amovible dans, et jusqu'au bout du canal central hermétique **28** de la sonde, l'extrémité, de la fibre optique **50,** avantageusement préalablement clivée, étant ainsi en contact avec le bout canal central. Le fibre **50** peut donc émettre le rayonnement électromagnétique au niveau de la portion de la sonde **16** destinée à la stimulation électrique du cerveau, et notamment au moins au travers du bouchon **30.**

Notamment dans le cadre du traitement de la maladie de Parkinson, et comme illustré à la figure 7B, le canal central **28** se prolonge au-delà de la dernière électrode de stimulation électrique **20d** sur une distance *d* suffisante pour que l'extrémité distale du canal, et donc le bouchon **30** au-travers duquel est réalisée l'illumination, soit positionnée dans la SNc du patient alors que l'électrode **20d** est positionnée dans la STN du patient. Notamment la distance d est comprise à cet effet entre 15mm et 20mm.

De manière avantageuse, la longueur *e* du bouchon **30** émettant de la lumière est comprise entre 2 et 5 mm pour illuminer localement la portion SNc du *substancia nigra.* Une longueur de quelques mm de diffusion de la lumière permet d'améliorer l'uniformité de l'éclairement d'une grande surface.

La fibre optique **50** est par exemple une fibre HCS (« *hard-clad silica* ») multimode ayant un cœur **56** de diamètre inférieure à 500 micromètres, par exemple compris entre 125 et 300 micromètres, enrobé d'une gaine **58,** par exemple une fibre optique de référence K200/230 Polymer Cladded Fiber de la société LEONI FIBER OPTICS. De telles fibres ont l'avantage d'être biocompatibles et permettent en outre un guidage optique de qualité, même lorsqu'elles sont courbées.

Avantageusement, la fibre optique peut subir un traitement mécanique, par exemple la gaine **58** peut être traité par un sablage, sur une portion distale ou localisée le long de la fibre, de manière à former des trous mettant à nu son cœur **56,** ce qui permet d'augmenter la surface d'émission de la fibre, ou découpler une partie de la lumière localement et notamment permettre à un pourcentage déterminé du flux de lumière d'être émis entre les espaces inter-électrode de la sonde **16.** D'autres solutions telles que le marquage laser de µ structures à l'intérieur de la fibre permettent d'obtenir le même effet d'extraction localement d'un pourcentage de la puissance lumineuse.

Pour la connexion électrique des contacts **22a, 22b, 22c, 22d,** un boitier électrique **60,** tel qu'illustré à la figure 8, est prévu. Le boitier **60** comprend un corps **62** électriquement isolant et réalisé en un matériau biocompatible, traversé de part en part par un canal cylindrique **64** de diamètre légèrement inférieur au diamètre de la partie proximale de la sonde **16.** Le canal **64** comprend des contacts électriques cylindriques **66a, 66b, 66c, 66d** définissant par exemple une surface interne de canal **64,** et dont les dimensions et l'espacement correspondent aux dimensions et espacement des contacts **22a, 22b, 22c, 22d.** Ainsi lorsque la portion proximale de la sonde **16** est introduite dans le canal **64,** les contacts de la sonde et du canal viennent respectivement en contact, la sonde étant ensuite bloquée en position par une pièce de blocage **68** du boitier **60.** Les éléments venant d'être décrits correspondent par exemple au modèle 37083 d'extension fabriqué par la société Medtronics. Le boitier **60** a une fonction de reprise de contact et ne comprend aucun élément de commande (microprocesseur, batterie, etc...), ces éléments étant contenus dans le stimulateur **40.**

De manière avantageuse, des fils conducteurs **70a, 70b, 70c, 70d** sont connectés respectivement aux contacts **66a, 66b, 66c, 66d** et logés dans l'épaisseur du corps **62.** Ceci permet de réaliser une rallonge électrique **72,** qui est fixée au boitier **60** tout en n'obstruant pas l'ouverture **74** du canal **64,** opposée à l'ouverture **76** par laquelle est insérée la sonde DBS. Le boitier **60** et la rallonge **72** forment ainsi une extension électrique pour la connexion de la sonde DBS à un stimulateur, qui permet l'insertion de la fibre optique dans le canal central de la sonde DBS et son raccordement à une connectique optique.

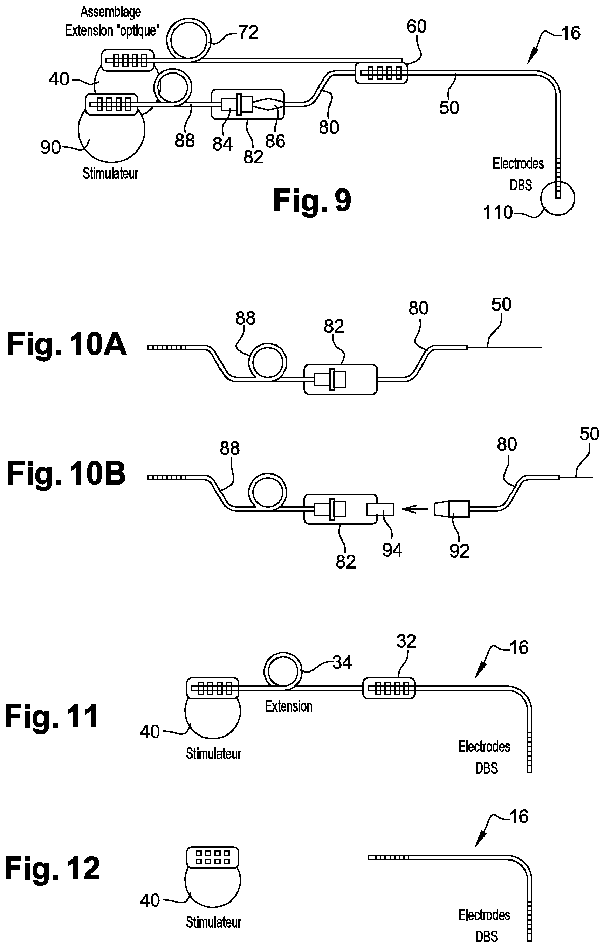

En se référant à la figure 9, la connexion de la sonde DBS et de la fibre optique insérée dans celle-ci, comprend :
- pour la stimulation électrique profonde, l'extension électrique **60, 72** connectée à un stimulateur électrique **40** ;
- pour la stimulation optique profonde, une rallonge optique formant guide d'onde **80** reprenant axialement la fibre optique **50** dans le canal du boitier de connexion électrique **60,** un boitier émetteur **82** auquel est raccordée la rallonge **80** et comprenant une source d'émission **84** apte à injecter dans la rallonge **80** le rayonnement **86** à la longueur d'onde d'intérêt, notamment à 670 nanomètres. Par exemple, la source d'émission **84** comprend une diode laser (par exemple une diode laser de référence HL6756MG de la société Thorlabs Inc.), une LED (« *Light-Emitting Diode* », par exemple une LED de référence SMT670 de la société Epitex), une OLED (« *Organic Light-Emitting Diode* ») ou un VCEL (« *vertical-cavity surface-emitting laser* »). Une rallonge électrique **88** fixée au boitier émetteur **80,** est également prévue pour la connexion de la source d'émission **84** avec un stimulateur optique **90,** qui contrôle le fonctionnement de cette dernière.

La rallonge électrique **80** et la fibre optique **50** comprennent par exemple à leur extrémité respective de raccordement, des connectiques permettant la reprise optique de fibres, comme cela est connu en soi de l'état de la technique. Par exemple, une portion proximale de la fibre **50** est collée dans un canal concentrique usiné dans une férule en céramique ou en acier calibrée en diamètre (e.g. de diamètres standards entre 1,25 et 2,5mm) pour garantir une bonne répétabilité de positionnement lors de la connexion dans une traversée optique (réceptacle) de couplage sur une source laser.

Avantageusement, le stimulateur optique **90** est un neuro-stimulateur classique, e.g. un stimulateur de structure identique au stimulateur **40** utilisé pour la stimulation électrique. En effet, comme cela est connu en soi, ce type de stimulateur délivre de façon programmable des signaux de commande et des tensions/courants d'alimentation électrique sur des bornes auxquelles est raccordée la rallonge électrique **72.** Un stimulateur peut ainsi être programmé pour envoyer des signaux sur les électrodes alternativement ou en même temps que la commande de la source lumineuse **84.**

En variante, un seul stimulateur est prévu pour commander la stimulation électrique et optique, ce stimulateur étant équipé d'une connectique pour la rallonge électrique **72** et d'une connectique pour la rallonge optique **80.**

Le stimulateur optique **90** commande notamment la puissance optique de la source, de manière à obtenir sur la portion distale de la sonde DBS, la puissance optique souhaitée. Plus particulièrement, pour une longueur d'onde de 670 nm, la puissance est sélectionnée inférieure à 10mW, ce qui permet de limiter l'échauffement de la zone de cerveau, comme cela est décrit dans le document « High Resolution MR-Thermometry is a proper method to characterize thermal safety of chronic implantable medical device », Reinhart, F., et al, Society for Neuroscience Annual Meeting, novembre 2014.

Les éléments venant d'être décrits sont constitués de matériaux biocompatibles. Par exemple, les matériaux électriquement isolants sont en silicone, en polyuréthane, en Kapton®, etc, et les matériaux conducteurs électriques sont en alliage de nickel-cobalt-chromium-molybdenum, notamment alliage vendu sous la référence « MP35N® » par la société Carpenter Technology Corp., Pennsylvanie, USA.

De manière avantageuse, le stimulateur optique **90** comprend un circuit de protection contre les décharges électriques statiques, ou circuit « ESD » (« *Electrostatic Discharge* »), en particulier lorsque la source d'émission **84** est une diode laser, un VCSEL, ou une microLED qui sont sensibles aux décharges électriques.

En se référant aux figures 10A et 10B, selon une première variante (figure 10A), la rallonge **80** connectant optiquement le boitier émetteur **82** à la fibre optique **50** est fixée de manière inamovible au boitier **82.** Selon une seconde variante (figure 10B), la rallonge est fixée de manière amovible au boitier **82,** et comprend par exemple un embout **92** venant s'enficher de manière hermétique aux fluides dans une embase complémentaire **94** du boitier **80,** ce qui permet de faciliter une procédure d'assemblage lors de la chirurgie, et de réaliser un test du bon fonctionnement / positionnement de la fibre optique lorsqu'elle est insérée dans la sonde DBS, comme cela sera décrit ci-après.

Il est à présent décrit en relation avec les figures 11 à 15 un procédé de mise à jour d'une paire de sondes DBS déjà implantées dans un cerveau, par exemple d'un patient humain, afin de leur conférer la fonction d'éclairement.

Sur la figure 11, une sonde DBS **16** est illustrée telle qu'implantée dans le patient, et donc raccordée à l'extension électrique **32, 34,** et au stimulateur **40.** Le procédé consiste donc, dans une première étape, à opérer le patient afin de pouvoir retirer l'extension électrique **32, 34** et le stimulateur **40** si ce dernier n'est pas compatible avec la mise à jour optique (figure 12), tout en laissant en place la sonde DBS **16.**

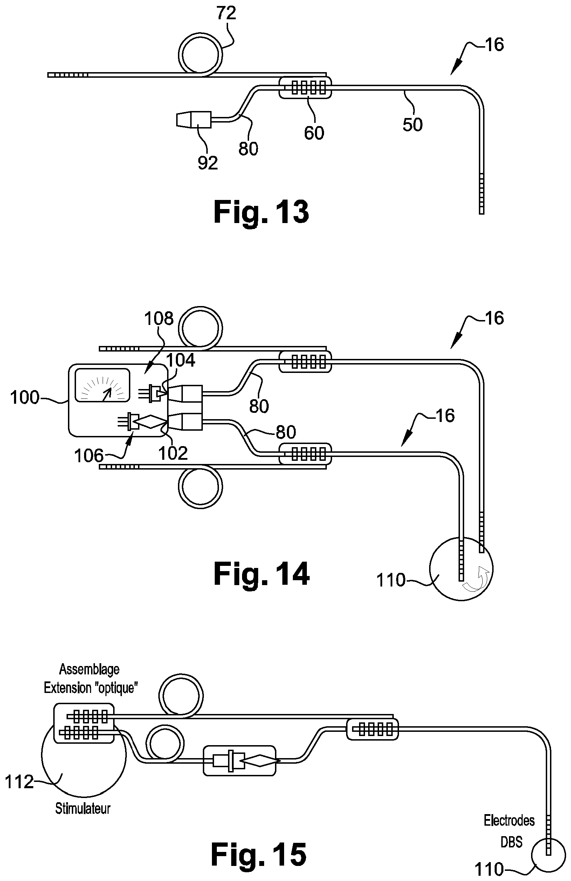

Dans une étape suivante (figure 13), la portion distale de la sonde DBS est introduite dans le canal central d'un boitier électrique **60** selon l'invention et bloquée en position. Une fibre optique **50** est ensuite insérée dans le canal central de la sonde DBS **16** au travers du canal du boitier **60,** jusqu'à ce que la fibre atteigne l'extrémité distale de la sonde. Une rallonge optique **60,** avantageusement la variante amovible décrite en relation avec la figure 10B, est alors raccordée à la fibre optique **50** ou bien est déjà assemblée avec la fibre optique **50** lors de son insertion dans le canal central de la sonde DBS.

Une fois cette opération réalisée pour les deux sondes DBS implantées, un procédé de test de leur bon fonctionnement optique est alors mis en œuvre. Plus particulièrement (figure 14), les rallonges optiques **80** des deux sondes DBS **16** sont raccordées à une première et une seconde bornes **102, 104** d'un dispositif de test **100.** Ce dernier comprend une source d'émission **106** permettant d'injecter le rayonnement à la longueur d'onde du traitement dans la rallonge optique raccordée à la première entrée **102,** et un circuit de détection optique **108,** par exemple à base de la photodiode, qui mesure la quantité de rayonnement reçu par la rallonge **80** raccordée à la seconde entrée **104.**

Le rayonnement émis par la sonde DBS raccordée à la première entrée **102** diffuse donc au travers de la zone du cerveau traitée **110,** et une partie du rayonnement diffusé est collectée par la fibre optique insérée dans l'autre sonde DBS. Le rayonnement ainsi collecté est transporté jusqu'au circuit de détection du dispositif de test **100,** où il est mesuré. Une fois cette mesure réalisée, la connexion des rallonges **80** au dispositif de test **100** est inversée pour procéder à une deuxième mesure, puis les deux mesures obtenues sont analysées pour déterminer le bon fonctionnement optique des sondes DBS. Par exemple, il est jugé que ce fonctionnement est satisfaisant lorsque chacune des mesures est supérieure à un seuil prédéterminée. Avantageusement, cette analyse est réalisée automatiquement par le circuit de détection optique **108** qui comprend à cet effet une unité de traitement à base de microprocesseur.

Si le bon fonctionnement optique des sondes DBS est établi, le procédé de mise à jour de ces dernières se poursuit (figure 15) par le remplacement du stimulateur électrique **40** par un stimulateur **112** configuré à la fois pour la stimulation optique et la stimulation électrique, les rallonges électrique et optique **72, 80** sont raccordées au stimulateur **112,** et ce dernier est mis en marche.

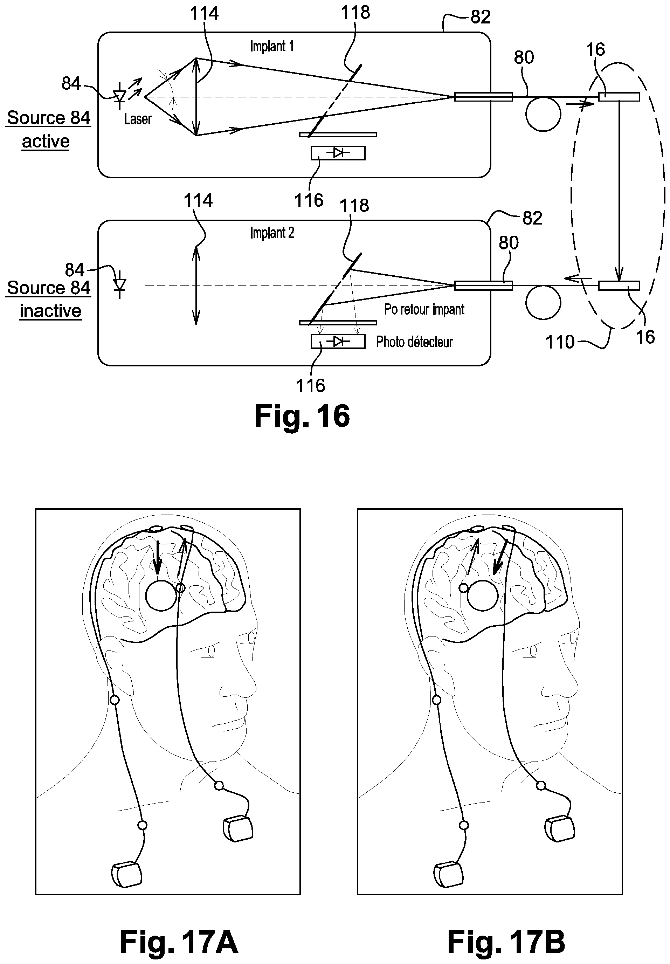

En se référant à la figure 16, qui illustre de manière simplifiée une paire de sondes de stimulation opto-électrique selon l'invention et leur boitier émetteur respectif **82,** le boitier émetteur **82** de chacune des sondes met également en œuvre une fonction de détection du rayonnement électromagnétique. Notamment, le boitier **82** comprend une source émettrice **84,** une optique **114** focalisant le rayonnement de la source **84** sur la rallonge optique **80** pour injecter le rayonnement dans celle-ci, un photo-détecteur **116** et un miroir semi-transparent. Le miroir laisse passer du rayonnement émis par la source **84,** comme illustré en partie haute de la figure 16, et dévie une partie du rayonnement en provenance de la rallonge **80** vers le photo détecteur **116,** comme cela est illustré en partie basse de la figure 16. En variante, la fibre optique a une ouverture numérique (e.g. égale à 0,37) supérieure à l'angle d'injection dans la rallonge (e.g. égal à 0,2). Le faisceau émis par la rallonge **80** est ainsi plus large que le faisceau focalisé sur la rallonge **80.** Le boitier **82** comporte alors un miroir **118,** par exemple annulaire, n'entravant pas le faisceau lumineux focalisé sur la rallonge optique **80** tout en étant situé dans le faisceau émis par la rallonge **80.** La portion du faisceau émis incident sur le miroir 118 est alors redirigée vers le photodétecteur **116.**

Le photodétecteur **116** est par ailleurs connecté au stimulateur **112** au travers de la rallonge **88** connectant le boitier **82** au stimulateur **112.** Ce dernier est alors configuré pour traiter la mesure délivrée par le photodétecteur **116** pour déterminer si le rayonnement détecté est conforme à un bon fonctionnement.

Ainsi, en désactivant l'éclairement d'une sonde, il est possible de tester le bon fonctionnement de l'autre sonde, d'une manière analogue à celle décrite en relation avec la figure 14, et de faire de même pour l'autre sonde, tel qu'illustré aux figures 18A et 18B. Le bon fonctionnement optique des sondes peut ainsi être testé à tout moment chez le patient, de manière automatique, et sans recourir à une quelconque opération chirurgicale.

Il a été décrit des modes de réalisation particuliers de l'invention. Bien entendu de multiples variantes sont possibles, en particulier concernant les connexions, les rallonges, etc...

Notamment, les caractéristiques suivantes peuvent être introduites, seules ou en combinaison :
- pour limiter les pertes de lumière et le risque de casse du guide d'onde (e.g. fibre optique), un "Stop sonde" imposant un rayon de courbure minimum à la sonde dans sa portion proximale placée au niveau de la trépanation ;
- un outillage de protection du guide d'onde en sortie de l'embase optique-électrique, et servant au centrage entre la fibre et le canal central de la sonde DBS pendant la mise en place du dispositif est utilisé ;
- pour faciliter l'insertion de la fibre optique, et particulièrement le passage d'un coude, on réalise un bout hémisphérique, par exemple une boule, à l'extrémité distale de la fibre par le dépôt d'une colle réticulable par irradiation UV suivi de la réticulation UV. Cette opération est réalisable à l'aide d'un outillage compatible avec une intervention en bloc chirurgical dans le cas où on ait besoin d'ajuster précisément la longueur de la sonde par clivage de la fibre qui laisse une terminaison droite et donc ne facilite pas l'insertion dans un canal. L'outillage permet un dépôt automatique de la colle et une réticulation "sécurisée" par arc électrique (soudeuse fibre) ;
- préalablement à l'insertion de la fibre optique dans la sonde DBS, il est procédé à l'injection dans le canal central de la sonde d'un silicone liquide, implantable sur le long terme, de fort indice de réfraction, qui réduit les pertes par réflexion en bout de sonde et limiter le risque d'infiltration de liquides physiologiques. L'invention est définie dans les revendications qui suivent. Les modes de réalisation décrits ci-dessus, qui sont soit incompatibles avec au moins l'une des revendications indépendantes ci-dessous, ou qui ne comportent pas l'ensemble des caractéristiques d'au moins l'une desdites revendications indépendantes ci-dessous, sont exclus de la présente invention.

## Revendications

1. Dispositif pour la stimulation profonde d'un cerveau, comprenant :
- une sonde de stimulation (16) comprenant :
∘ un corps longiligne (18) parcouru par un canal central vide de matière (28) s'étendant entre une portion distale et une extrémité proximale de la sonde (16), le canal central (28) étant ouvert à ladite extrémité proximale et la portion distale du corps (18) étant apte à transmettre une longueur d'onde prédéterminée ;
∘ des électrodes (20a, 20b, 20c, 20d) fixées sur une surface externe du corps (18) dans la portion distale de la sonde pour la stimulation électrique du cerveau ; et
∘ des bornes électriques (22a, 22b, 22c, 22d) fixées sur une surface externe du corps (18) dans la portion proximale de la sonde et connectées aux électrodes (20a, 20b, 20c, 20d) pour leur alimentation électrique;
- un boitier électrique (60) comprenant un canal (64) dans lequel sont logées des bornes électriques (66a, 66b, 66c, 66d), ledit canal (64) étant ouvert à l'une de ses extrémité (76) pour recevoir la portion proximale de la sonde de manière à mettre en contact les bornes électriques (22a, 22b, 22c, 22d) de la sonde et les bornes électriques (66a, 66b, 66c, 66d) du boitier électrique (60); le canal (64) du boîtier électrique (60) étant ouvert à une deuxième extrémité (74), opposée à l'ouverture (76) par laquelle est insérée la portion proximale de la sonde;
- une rallonge électrique (72) fixée au boitier électrique (60) et connectée aux bornes électriques (66a, 66b, 66c, 66d) du boitier (60); dispositif comportant en outre :
∘ un guide d'onde (50) apte à guider la longueur d'onde prédéterminée, logé au moins en partie dans le canal central (28) du corps (18) de la sonde, s'étendant jusqu'à la portion distale de la sonde pour l'illumination du cerveau au travers de celle-ci; et
∘ un boitier émetteur (82) apte à être raccordé à l'extrémité proximale du guide d'onde (50) pour l'injection dans celui-ci de la longueur d'onde prédéterminée; dispositif dans lequel:
- le canal (64) est cylindrique avec un diamètre légèrement inférieur au diamètre de la portion proximale de la sonde (16) de sorte que, lorsque la portion proximale de la sonde (16) est introduite dans le canal (64) par l'extrémité (76), les bornes électriques (22a, 22b, 22c, 22d) de la sonde (16) viennent respectivement en contact des bornes électriques (66a, 66b, 66c, 66d) du boîtier électrique (60); et dans lequel:
- ladite rallonge électrique (72) est fixée au boîtier (82) sans obstruer la deuxième extrémité (74) du canal (64) du boîtier .

2. Dispositif pour la stimulation profonde d'un cerveau selon la revendication 1, dans lequel la portion distale du guide d'onde et la portion distale de la sonde ont une longueur comprise entre 2 millimètres et 5 millimètres.

3. Dispositif pour la stimulation profonde d'un cerveau selon la revendication 1 ou 2, dans lequel la longueur d'onde comprise entre 670nanomètres et 1070 nanomètres, et de préférence entre 670 nanomètres et 810 nanomètres, et en ce que la puissance optique émise par la portion distale du guide d'onde est inférieure ou égale à 10 milliwatts.

4. Dispositif pour la stimulation profonde d'un cerveau selon la revendication 1, 2 ou dans lequel le guide d'onde est une fibre optique de type « hard-clad silica» ayant un coeur silice de diamètre compris entre 125 micromètres et 300 micromètres.

5. Extension électrique (60, 72) pour une connexion avec une sonde de stimulation profonde d'un cerveau (16), ladite sonde comprenant 2. un corps longiligne (18) parcouru par un canal central vide de matière (28), apte à recevoir un guide d'onde (50), et s'étendant entre une portion distale et une extrémité proximale de la sonde (16), le canal central (28) étant ouvert à ladite extrémité proximale et la portion distale du corps (18) étant apte à transmettre une longueur d'onde prédéterminée; des électrodes (20a, 20b, 20c, 20d) fixées sur une surface externe du corps (18) dans la portion distale de la sonde pour la stimulation électrique du cerveau; et des bornes électriques (22a, 22b, 22c, 22d) fixées sur une surface externe du corps (18) dans la portion proximale de la sonde et connectées aux électrodes (20a, 20b, 20c, 20d) pour leur alimentation électrique; ladite extension électrique comprenant
- un boitier électrique (60) comprenant :
∘ un canal ouvert (64) à une première et une seconde extrémités, (76) le canal (64) étant apte à recevoir la portion proximale du corps de la sonde lorsque celle-ci est introduite par la première extrémité du canal; le canal (64) du boîtier électrique (60) étant également ouvert à une deuxième extrémité (74), opposée à l'ouverture (76) par laquelle est insérée la portion proximale de la sonde;
∘ des bornes électriques (66a, 66b, 66c, 66d) logées dans le canal (64) du boitier électrique (60) et aptes à venir au contact des bornes (22a, 22b, 22c, 22d) de la sonde lorsque la portion proximale de celle-ci est introduite dans le canal (64) du boitier (60) par la première extrémité du canal; une rallonge électrique (72) comprenant des conducteurs électriques (70a, 70b, 70c, 70d) raccordés aux bornes électriques (66a, 66b, 66c, 66d) du boîtier électrique; extension dans laquelle
∘ le canal (64) est cylindrique avec un diamètre légèrement inférieur au diamètre de la portion proximale de la sonde (16) de sorte que, lorsque la portion proximale de la sonde (16) est introduite dans le canal (64), les bornes électriques (22a, 22b, 22c, 22d) de la sonde (16) viennent respectivement en contact des bornes électriques (66a, 66b, 66c, 66d) du boitier électrique (60); et dans laquelle ladite rallonge électrique est fixée au boitier sans obstruer la deuxième extrémité (74) du canal du boitier.

6. Procédé de test du fonctionnement optique d'un premier et d'un deuxième dispositifs de stimulation selon l'une quelconque des revendications 1 à 4, les sondes des deux dispositifs étant pré-implantées dans le cerveau avec leur portion distale logées dans une zone du cerveau à traiter, le procédé consistant :
- à injecter un rayonnement d'une longueur d'onde prédéterminée dans le guide d'onde du premier dispositif par l'extrémité proximale du guide d'onde et à mesurer ledit rayonnement à l'extrémité proximale du guide d'onde du deuxième dispositif;
- à injecter un rayonnement de même longueur d'onde dans le guide d'onde du deuxième dispositif par l'extrémité proximale du guide d'onde et à mesurer ledit rayonnement à l'extrémité proximale du guide d'onde du premier dispositif; et
- à diagnostiquer le fonctionnement optique de l'ensemble formé des sondes et des guides d'onde logés dans celle-ci en fonction des mesures.

## Patentansprüche

1. Vorrichtung für tiefe Hirnstimulation, mit:
- einer Stimulationssonde (16), die besteht aus::
∘ einem länglichen Körper (18), durch den ein zentraler, materialfreier Kanal (28) verläuft, der sich zwischen einem distalen Abschnitt und einem proximalen Endstück der Sonde (16) erstreckt, der zentrale Kanal (28) ist zu diesem proximalen Endstück hin offen und der distale Abschnitt des Körpers (18) ist in der Lage, eine vorher festgelegte Wellenlänge zu übermitteln;
∘ Elektroden (20a, 20b, 20c, 20d), befestigt auf einer Außenfläche des Körpers (18) im distalen Abschnitt der Sonde zur elektrischen Stimulation des Hirns; und
∘ elektrische Anschlüsse (22a, 22b, 22c, 22d), befestigt an einer Außenfläche des Körpers (18) im proximalen Abschnitt der Sonde und verbunden mit den Elektroden (20a, 20b, 20c, 20d) zur Stromversorgung;
- einem Schaltkasten (60) mit einem Kanal (64), in dem die elektrischen Anschlüsse (66a, 66b, 66c, 66d) untergebracht sind, dieser Kanal (64) ist an einem seiner Enden (76) offen, zur Aufnahme des proximalen Abschnitts der Sonde, so dass die elektrischen Anschlüsse (22a, 22b, 22c, 22d) der Sonde und die elektrischen Anschlüsse (66a, 66b, 66c, 66d) des Schaltkastens (60) in Kontakt gebracht werden; der Kanal (64) des Schaltkastens (60), ist dabei an einem zweiten Ende (74), gegenüber der Öffnung (76) offen, durch die der proximale Abschnitt der Sonde eingeführt wird;
- ein Verlängerungskabel (72), befestigt am Schaltkasten (60) ist mit den elektrischen Anschlüssen (66a, 66b, 66c, 66d) des Kastens (60) verbunden;
die Vorrichtung enthält außerdem:
∘ einen Wellenleiter (50), der in der Lage ist, die vorher festgelegte Wellenlänge zu leiten und zumindest zum Teil im zentralen Kanal (28) des Körpers (18) der Sonde untergebracht ist, der bis zum distalen Abschnitt der Sonde verläuft, um das Hirn durch diese zu beleuchten; und
∘ ein Sendergehäuse (82), das mit dem proximalen Ende des Wellenleiters (50) verbunden werden kann, um in diesen die vorher festgelegte Wellenlänge einzuspeisen; die Vorrichtung ist **dadurch gekennzeichnet, dass**:
- der Kanal (64) zylinderförmig ist, mit einem Durchmesser, der etwas kleiner ist, als der Durchmesser des proximalen Abschnitts der Sonde (16), so dass, wenn der proximale Abschnitt der Sonde (16) in den Kanal (64) über das Ende (76) eingeführt wird, die elektrischen Anschlüsse (22a, 22b, 22c, 22d) der Sonde (16) jeweils in Kontakt mit den elektrischen Anschlüssen (66a, 66b, 66c, 66d) des Schaltkastens (60) kommen; und dadurch, dass
- das erwähnte Verlängerungskabel (72) am Gehäuse (82) befestigt ist, ohne das zweite Ende (74) des Kanals (64) des Gehäuses zu blockieren.

2. Vorrichtung für tiefe Hirnstimulation nach Anspruch 1, ***dadurch gekennzeichnet, dass*** der distale Abschnitt des Wellenleiters und der distale Abschnitt der Sonde eine Länge zwischen 2 Millimetern und 5 Millimetern haben

3. Vorrichtung für tiefe Hirnstimulation nach Anspruch 1 oder 2, ***dadurch gekennzeichnet, dass*** die Wellenlänge zwischen 670 Nanometern und 1070 Nanometern liegt, und vorzugsweise zwischen 670 Nanometern und 810 Nanometern, und ***dadurch, dass*** die optische Leistung, die vom distalen Abschnitt des Wellenleiter ausgesendet wird, kleiner oder gleich 10 Milliwatt ist.

4. Vorrichtung für tiefe Hirnstimulation nach Anspruch 1, 2 oder 3, ***dadurch gekennzeichnet, dass*** es sich bei dem Wellenleiter um eine optische Faser vom Typ « Hard Clad Silica » handelt, mit einem Kern aus Silizium mit einem Durchmesser zwischen 125 Mikrometern und 300 Mikrometern.

5. Elektrische Erweiterung (60, 72) für eine Verbindung mit einer Sonde für die tiefe Hirnstimulation (16), die erwähnte Sonde enthält einen länglichen Körper (18), durch den ein zentraler, materialfreier Kanal (28) verläuft, der einen Wellenleiter (50) aufnehmen kann, und sich zwischen einem distalen Abschnitt und einem proximalen Endstück der Sonde (16) erstreckt, der zentrale Kanal (28) ist zu diesem proximalen Endstück hin offen und der distale Abschnitt des Körpers (18) ist in der Lage, eine vorher festgelegte Wellenlänge zu übermitteln; Elektroden (20a, 20b, 20c, 20d), befestigt auf einer Außenfläche des Körpers (18) im distalen Abschnitt der Sonde zur elektrischen Stimulation des Hirns; und elektrische Anschlüsse (22a, 22b, 22c, 22d), befestigt an einer Außenfläche des Körpers (18) im proximalen Abschnitt der Sonde und verbunden mit den Elektroden (20a, 20b, 20c, 20d) zur Stromversorgung; diese elektrische Erweiterung umfasst:
- einen Schaltkasten (60) mit:
∘ einem Kanal (64), der zu einem ersten und einem zweiten Ende (76) hin offen ist, der Kanal (64) kann dabei den proximalen Abschnitt des Körpers der Sonde aufnehmen, wenn diese durch das erste Ende des Kanals eingeführt wird; der Kanal (64) des Schaltkastens (60) steht auch gegenüber der Öffnung (76), durch die der proximale Abschnitt der Sonde eingeführt wird, zu einem zweiten Ende (74) hin offen;
∘ elektrische Anschlüsse (66a, 66b, 66c, 66d), untergebracht im Kanal (64) des Schaltkastens (60) und die in Kontakt mit den elektrischen Anschlüssen (66a, 66b, 66c, 66d) der Sonde kommen können, wenn ihr proximaler Abschnitt in den Kanal (64) des Gehäuses (60) über das erste Ende des Kanals eingeführt wird; ein Verlängerungskabel (72) mit Elektroleitungen (70a, 70b, 70c, 70d), verbunden mit den elektrischen Anschlüssen (66a, 66b, 66c, 66d) des Schaltkastens, diese elektrische Erweiterung ist **dadurch gekennzeichnet, dass**
∘ der Kanal (64) zylinderförmig ist, mit einem Durchmesser, der etwas kleiner ist, als der Durchmesser des proximalen Abschnitts der Sonde (16), so dass, wenn der proximale Abschnitt der Sonde (16) in den Kanal (64 (76) eingeführt wird, die elektrischen Anschlüsse (22a, 22b, 22c, 22d) der Sonde (16) jeweils in Kontakt mit den elektrischen Anschlüssen (66a, 66b, 66c, 66d) des Schaltkastens (60) kommen;
und dadurch, dass
∘ das erwähnte Verlängerungskabel am Gehäuse befestigt ist, ohne das zweite Ende (74) des Kanals des Gehäuses zu blockieren.

6. Verfahren zum Test der optischen Funktion einer ersten und zweiten Stimulationsvorrichtung nach einem beliebigen der Ansprüche 1 bis 4, die Sonden der beiden Vorrichtungen sind im Hirn vorimplantiert, wobei ihr distaler Abschnitt in einem zu behandelnden Hirnbereich untergebracht ist, das Verfahren besteht dabei darin:
- eine Strahlung mit einer vorher festgelegten Wellenlänge in den Wellenleiter der ersten Vorrichtung des Wellenleiters einzuspeisen und diese Strahlung am proximalen Ende des Wellenleiters der zweiten Vorrichtung zu messen;
- eine Strahlung mit derselben Wellenlänge in den Wellenleiter der zweiten Vorrichtung des Wellenleiters einzuspeisen und diese Strahlung am proximalen Ende des Wellenleiters der zweiten Vorrichtung zu messen; und
- die optische Funktion der Einheit aus Sonden und in diesen untergebrachten Wellenleitern entsprechend der Messungen zu diagnostizieren.

## Claims

1. A deep brain stimulation device comprising:
- a stimulation probe (16) comprising:
▪ a longilineal body (18) having a central channel void of material (28) running therethrough, extending between a distal portion and a proximal end of the probe (16), the central channel (28) being open at said proximal end and the distal portion of the body (18) being capable of transmitting a predetermined wavelength;
▪ electrodes (20a, 20b, 20c, 20d) affixed to an outer surface of the body (18) in the distal portion of the probe for electric brain stimulation; and
▪ electrical terminals (22a, 22b, 22c, 22d) affixed to an outer surface of the body (18) in the proximal end of the probe and connected to the electrodes (20a, 20b, 20c, 20d) for their electric power supply;
- an electrical housing (60) comprising a channel (64) having electrical terminals (66a, 66b, 66c, 66d) housed therein, said channel (64) being open at one of its ends (76) to receive the proximal portion of the probe to place into contact the electrical terminals (22a, 22b, 22c, 22d) of the probe and the electrical terminals (22a, 22b, 22c, 22d) of the housing (60); the channel (64) of the electrical housing (60) being open at a second end (74), opposed to first end (76), through which is inserted the proximal portion of the probe ,
- an electrical extension lead (72) affixed to the electrical housing (60) and connected to the electrical terminals (66a, 66b, 66c, 66d) of the housing (60),
said device further comprising :
- a waveguide (50) capable of guiding the predetermined wavelength, housed at least partly in the central channel (28) of the probe body (18), and extending all the way to the distal portion of the probe for the illumination of the brain therethrough; and
- a transmitter housing (82) capable of being connected to the proximal end of the waveguide (50) for the injection into it of the predetermined wavelength; the device being **characterized :**
- **in that** the channel (64) is cylindrical and having a diameter slightly smaller than the diameter of the proximal portion of probe (16), so that when the proximal portion of the probe (16) is inserted into the channel (64) through the end (76), the electrical terminals (22a, 22b, 22c, 22d) of the housing (60) of the probe (16) come respectively into contact with the electrical terminals (66a, 66b, 66c, 66d) of the housing (60);
- and **in that** said electrical extension lead (72) is affixed to the housing (82) without obstructing the second end (74) of the channel (64) of the housing.

2. The deep brain stimulation device of claim 1, ***characterized* in that** the distal portion of the waveguide and the distal portion of the probe have a length in the range from 2 millimeters to 5 millimeters.

3. The deep brain stimulation device of claim 1 or 2, ***characterized* in that** the wavelength is in the range from 670 nanometers to 1,070 nanometers, and preferably from 670 to 810 nanometers, and **in that** the optical power transmitted by the distal portion of the waveguide is smaller than or equal to 10 milliwatts.

4. The deep brain stimulation device of claim 1, 2, or 3, ***characterized* in that** the waveguide is a "hard-clad silica" type optical fiber having a silica core with a diameter in the range from 125 micrometers to 300 micrometers.

5. An electrical extension lead (60, 72) for a connection with a deep brain stimulation probe (16), said probe comprising a longilineal body (18) crossed by a central channel (28) void of material, and able to receive a waveguide (50), and extending between a proximal portion and a distal portion of the probe (16), said central channel (28) being open at said proximal portion and the distal end of the body (18) being capable of transmitting a predetermined wavelength ; electrodes (20a, 20b, 20c, 20d) affixed to an outer surface of the body (18) in the distal portion of the probe for electric brain stimulation ; and electrical terminals (22a, 22b, 22c, 22d) affixed to an outer surface of the body (18) in the proximal portion of the probe body and connected to the electrodes (20a, 20b, 20c, 20d) for their electric power supply, said electrical extension lead comprising:
- an electrical housing (60) comprising:
▪ a channel (64) open at a first and a second ends (76), the channel (64) being capable of receiving the proximal portion of the body of the probe when the latter is introduced through the first end of the channel; the channel (64) of the electrical housing (60) being also open at a second end (74), opposite to the first end (76) through which is inserted the proximal portion of the probe ;
▪ electrical terminals (66a, 66b, 66c, 66d) housed in the channel (64) of the electrical housing (60) and capable of coming into contact with the probe terminals (22a, 22b, 22c, 22d) when the proximal portion thereof is introduced into the channel (64) of the housing (60) through the first end of the channel;
- an electrical extension lead (72) comprising electrical conductors (70a, 70b, 70c, 70d) connected to the electrical terminals (66a, 66b, 66c, 66d) of the electrical housing, said electrical extension lead being characterized :
▪ in that the channel (64) is cylindrical with a diameter slightly smaller than the diameter of the proximal portion of probe (16), so that when the proximal portion of the probe (16) is inserted into the channel (64) through the end (76), the electrical terminals (22a, 22b, 22c, 22d) of the housing (60) of the probe (16) come respectively into contact with the electrical terminals (66a, 66b, 66c, 66d) of the housing (60);
▪ and in that said electrical extension lead (72) is affixed to the housing (82) without obstructing the second end (74) of the channel (64) of the housing.

6. A method of testing the optical operation of a first and a second stimulation devices of any of claims 1 to 4, the probes of the two devices being pre-implanted in the brain with their distal portions housed in an area of the brain to be treated, the method comprising:
- injecting a radiation having a predetermined wavelength into the waveguide of the first device through the proximal end of the waveguide and measuring said radiation at the proximal end of the waveguide of the second device;
- injecting a radiation having the same wavelength into the waveguide of the second device through the proximal end of the waveguide and measuring said radiation at the proximal end of the waveguide of the first device; and
- diagnosing the optical operation of the assembly formed by the probes and the waveguides housed therein according to the measurements.
